# EUROPEAN PATENT SPECIFICATION

(11) **EP 2 976 421 B1**
(45) Date of publication and mention of the grant of the patent: **09.11.2016**
(21) Application number: 14712200.6
(22) Date of filing: 19.03.2014
(51) Int. Cl.: C12M 1/107, C12M 1/00

(54) **A BIO-ELECTROCHEMICAL SYSTEM FOR REMOVING INHIBITORS OF ANAEROBIC DIGESTION PROCESSES FROM ANAEROBIC REACTORS**
BIO-ELEKTROCHEMISCHES SYSTEM ZUR BESEITIGUNG VON HEMMERN ANAEROBER VERDAUUNGSPROZESSE AUS ANAEROBEN REAKTOREN
SYSTÈME BIO-ÉLECTROCHIMIQUE POUR SUPPRIMER DES INHIBITEURS DE PROCESSUS DE DIGESTION ANAÉROBIE DE RÉACTEURS ANAÉROBIE

(30) Priority: 22.03.2013 EP 13160580
(43) Date of publication of application: 27.01.2016
(73) Proprietor: Danmarks Tekniske Universitet, 2800 Kgs. Lyngby (DK)
(72) Inventor: ANGELIDAKI, Irini, DK-2000 Frederiksberg (DK); ZHANG, Yifeng, 2800 Kongens Lyngby (DK)
(74) Representative: Plougmann Vingtoft a/s
(86) International application number: PCT/DK2014/050067
(87) International publication number: WO 2014/146671

(56) References cited:
- EP-A2- 0 301 924
- WO-A1-2008/110176
- WO-A1-2009/008709
- WO-A2-2008/107663
- WO-A2-2008/133742
- DE-A1-102009 037 946
- US-A1- 2004 045 885

## Description

### FIELD OF THE INVENTION

The present invention relates to a bio-electrochemical system and a method for removing inhibitors of anaerobic digestion processes, such as ammonia and sulphate, from anaerobic reactors. The invention further relates to an anaerobic digester and a plant for producing biogas comprising a bio-electrochemical system for removing inhibitors of anaerobic digestion processes.

### BACKGROUND OF THE INVENTION

Anaerobic digestion is a known process industrially used for example in the production of biogas.
Anaerobic digestion processes can be inhibited by the presence of several substances, such as ammonia and/or sulphate. Thus in general the presence of ammonia (NH⁴⁺/NH₃) and/or sulphate (SO₄²⁻) in anaerobic digesters is the most serious problem of biogas plants as it reduces the efficiency of the conversion processes involved in the anaerobic digestion. In addition, during anaerobic digestion sulphate can be reduced to H₂S that can cause corrosion problems, malodour and toxicity.
In particular, the presence of ammonia is the major inhibitor in full scale digester of animal waste rich in urea and/or proteins.
In general, no viable method to overcome this problem has been found up to now as conventional method of removing ammonia such as by chemical precipitation, electrodialysis and air or steam stripping are very energy intensive and inefficient and thus not suitable for the application in biogas production.
Hence, a method and a system to improve the efficiency of conversion to biogas in biogas production plant employing anaerobic digestion processes would be advantageous, and in particular a more efficient and/or reliable method and system to remove and recover ammonia and/or sulphuric acid from anaerobic digester would advantageous. WO2008107663 discloses a bio-electrochemical system for the removal of inhibitory products, such as ammonia or lactic acid.

### OBJECT OF THE INVENTION

It is an object of the invention to improve the efficiency of biogas production plant.
It may be seen as an object of the invention to provide a system and a method to remove and recover ammonia from anaerobic digester.
It may be seen as a further object of the invention to provide a system and a method to remove and recover sulphate from anaerobic digester.
It is a further object of the present invention to provide an alternative to the prior art.
In particular, it may be seen as an object of the present invention to provide a bio-electrochemical system that solves the above mentioned problems of the prior art by removing inhibitors of anaerobic digestion processes.

### SUMMARY OF THE INVENTION

This invention proposes an innovative submersible bio-electrochemical system according to claim 1 that increases the efficiency of biogas plant by enhancing the conversion of ammonia-rich wastes to biogas. This is achieved by alleviating and/or counteracting the ammonia inhibition through a process of ammonia removal and recovery.
Thus, the above described object and several other objects are intended to be obtained in a first aspect of the invention by providing a submergible bio-electrochemical system for recovering ammonia in an anaerobic digester, comprising: an anode chamber; a cathode chamber, wherein the cathode chamber comprises a fluid inlet, a fluid outlet, an opening, a cathode and a cation exchange membrane (CEM) located at the opening of the cathode chamber, and wherein the anode chamber comprises a fluid inlet, a fluid outlet, an opening, an anode and an anion exchange membrane (AEM) located at the opening of the anode chamber.
The CEM and AEM separate the cathode chamber and the anode chamber from the outside environment, respectively.
Submergible bio-electrochemical system is defined as suitable for being submerged in an anaerobic digester, thus resistant to that environment and being adapted to allow complete submersion. Thus submergible is defined as not only to be suitable to be submerged but also as designed, i.e. adapted, to operate under a liquid surface.

The bio-electrochemical system may be used to recover ammonia, i.e. to remove ammonia from an anaerobic digester environment.
Anaerobic digestion is known process and is widely used as a source of renewable energy, e.g. to produce biogas.
An anaerobic digester is thus defined as a reactor in which a series of processes where microorganisms break down biodegradable material in the absence of oxygen occur.
Ion exchange is an exchange of ions between two electrolytes or between an electrolyte solution and a complex.
Ion exchangers are either cation exchangers that exchange positively charged ions (cations) or anion exchangers that exchange negatively charged ions (anions). Thus a cation exchange membrane (CEM) is a membrane allowing for cation exchange or getting across between the liquid contained in the anaerobic digester and the fluid contained in the cathode chamber.
Accordingly the anion exchange membrane (AEM) is a membrane allowing for anion exchange or getting across between the liquid contained in the anaerobic digester and the fluid contained in the anode chamber.
When in operation the anode and cathode are connected through an external circuit. In this way when submerged the bio-electrochemical system produces electricity by oxidizing organic material at the anode, thus producing electrons at the anode which are collected through the external circuit and reach the cathode, where they enter the reduction of oxygen leading to the formation of hydroxyl anions.
The invention is thus particularly, but not exclusively, advantageous for removing and recovering ammonia from anaerobic digesters thus enhancing the conversion of ammonia-rich wastes to biogas and at the same time produce electricity.

In some embodiments the opening comprised in the anode chamber allows for contact between the liquid contained in the anaerobic digester and the AEM contained in the anode chamber, when the submergible bio-electrochemical system is submerged.
When the bio-electrochemical system is submerged the presence of an opening in the anode chamber allows for the flow of the liquid contained in the anaerobic digester towards the AEM thus anion exchange/flow can occur between the liquid contained in the anaerobic digester and the fluid contained in the anode chamber. For example chloride anions, Cl⁻ or sulphates anions, SO₄²⁻ may cross the AEM and enter the anode chamber.

In some other embodiments the opening comprised in the cathode chamber allows for contact between the liquid contained in the anaerobic digester and the CEM contained in the cathode chamber, when the submergible bio-electrochemical system is submerged.
When the bio-electrochemical system is submerged the presence of an opening in the cathode chamber allows for the flow of the liquid contained in the anaerobic digester towards the CEM thus cation exchange can occur between the liquid contained in the anaerobic digester and the fluid contained in the cathode chamber. For example, ammonium cations, NH₄⁺ may cross the CEM and enter the cathode chamber.

In some embodiments the anode and cathode chamber are separated from each other. For example the anode and cathode chambers, when submerged, may be located at a certain distance from each other. This may increase the versatility of the system.

In some further embodiments the anode and cathode chamber are mechanically joined to each other. The anode and cathode chambers may be separated and joined just before the submersion so as to allow a more compact and easier handling of the bio-electrochemical system.

In some embodiments the anode and cathode chambers are compartments of a single bio-electrochemical chamber, said compartments being separated by fluid tight separation means.
A single chamber may contain both the anode and cathode chambers separated from each other by fluid tight separation means, e.g. a plate, such as a polycarbonate plate. In these embodiments the cathode chamber and the anode chamber have at least one side, such as a wall, in common. The at least one side in common may be the fluid tight separation means.

In some embodiments, multiple anode or cathode electrodes are used to improve the recover rate and electricity generation.

In some embodiments the anode chamber is separated into two sections, an acid recovery section and an anode section comprising the anode, wherein the acid recovery section comprises the opening of the anode chamber and is separated from the anode section by a further membrane.

The further membrane may be a bipolar membrane or a further cation exchange membrane.
A bipolar membrane is formed of an anion and a cation exchange layer that are bound together, either physically or chemically, and a very thin interface where the water diffuses from the outside of the membrane. In both embodiments with a bipolar membrane or with a cation exchange membrane the function of this further membrane is to transport out of the anode section the protons produced by the oxidation at the anode of the organic material contained in the fluid of the anode chamber.

In some further embodiments the acid recovery section further comprises a second fluid inlet and a second fluid outlet.
The second fluid inlet allows an aqueous solution to enter the acid recovery section, while the second fluid outlet allows for collection of the sulphuric acid solution out of the acid recovery section.
The acid recovery section has the function of temporary storing the recovered acid, such as sulphuric acid removed for the liquid contained in the anaerobic digester. When submerged the liquid contained in the anaerobic digester will enter in contact with the AEM and the anions therein contained, such as sulphate anions, may enter the acid recovery section through anion exchange at the AEM. Protons from the anode section will also travel to the acid recovery section through the further membrane. In this way an acid solution, such as a sulphuric acid solution, will be recovered in the acid recovery section and can be collected through the second fluid outlet.

In some embodiments the anode section comprises the second fluid inlet and the second fluid outlet.

The acid recovery section may be detachable from the anode section, thus the anode chamber may be physically separated into the anode section and the acid recovery section.
The acid recovered in the acid recovery section may be e.g. sulphuric acid, H₂SO₄ or chloric acid, HCl. The acid recovered may be used to neutralize the ammonia removed and recovered from the cathode chamber. Thus, the presence of the acid recovery section has the further synergistic advantage of neutralizing the ammonia recovered through the use of the recovered acid further reducing the overall cost involved in the biogas production.

In some embodiments the anode comprises anaerobic microorganisms. Anaerobic microorganism may be any kind of bacteria with electrochemical activity and microbes operating in anaerobic conditions.

The idea of the invention is to remove inhibitors in the biogas production through the use of a bio-electrochemical system that at the same time can produce electricity. The anode and cathode of the bio-electrochemical system are connected so as to produce electricity while the system comprising CEM, AEM, the cathode chamber and the anode chamber eventually separated into an acid recovery section and anode section allows for removal of inhibitors.
In a preferred embodiment the anode comprises a bio-film of anaerobic microorganisms. In this preferred embodiment the invention takes advantage of the ability of microorganisms to convert the energy released in metabolic reactions into electrical energy. At the anode in the anaerobic chamber, anaerobic microorganism with electrochemical activity can transfer electrons to the anode directly or indirectly via mediator molecule, thus in practice the electron transport chain is transferred outside of the microorganism from which energy can be harvested.
The anaerobic microorganisms grow as a bio-film at the anode and break down food wastes and sewage to generate electrons.
Using anaerobic microorganisms to generate electricity implies that the process becomes self-sustaining; the microorganisms replicate and continue to produce power indefinitely as long as there is a food source to nourish them. Moreover, the bio-electrochemical system becomes very efficient and can run effectively on sources like waste and sewage.

In this embodiment the anode and cathode chamber may be referred to also as the anaerobic and aerobic chamber respectively. Indeed the anaerobic chamber comprising the anode preserves the anaerobic condition of the anaerobic digester allowing for conversion of the metabolic energy of the anaerobic microorganisms into electricity. The aerobic chamber comprising the cathode is kept in aerobic condition by supplying an oxygen containing gas, such as air, through a fluid inlet. The chambers are therefore separated by fluid tight means so that the anaerobic and aerobic condition can be kept for the two separate chambers.
When in operation the anaerobic microorganisms on the anode decompose organic matter and free protons and electrons are produced. The electrons flow from the anaerobic microorganisms to the anode and through a wire towards the cathode. While flowing through the wire, an electrical current is generated that can be used to perform work. The protons, H⁺, released in the anode chamber flow either to the acid recovery section through the appropriate membrane or, when the acid recovery section is not present, decrease the pH of the anode chamber and are removed together with the fluid present in the anode chamber through the fluid outlet of the anode chamber.
The electrons at the cathode combine with dissolved oxygen gas provided through the fluid inlet of the cathode chamber and the aqueous solution present in the cathode chamber leading to the formation of hydroxyl anions. These hydroxyl anions react in turn with the ammonium cations entering the cathode chamber through the CEM producing ammonia, NH₃, that is released through the fluid outlet of the cathode chamber.

In a second aspect the invention provides a method for removing ammonia in anaerobic digesters, the method comprises: submerging in anaerobic digesters a submergible bio-electrochemical system according to the first aspect of the invention; operating the bio-electrochemical system, thereby producing electricity and a stream of air and ammonia gas out of the fluid outlet of the cathode chamber.
Submerging the bio-electrochemical system in anaerobic digesters allows for the contact between the liquid contained in the anaerobic digester and the AEM and CEM.

The step of operating the bio-electrochemical system may comprise: introducing a waste fluid at the inlet of the anode chamber, such as waste influent to the anaerobic digester or waste effluent at the outlet of the anaerobic digester; introducing a stream of fluid at the inlet of the cathode chamber, such as air or a gas mixture containing oxygen gas; collecting an anode chamber effluent of treated waste fluid at the outlet of the anode chamber; collecting a cathode chamber effluent of air or a gas mixture containing ammonia gas at the outlet of the cathode chamber.

The step of operating the bio-electrochemical system may comprise treating the cathode chamber effluent by bubbling the cathode chamber effluent though an acid solution thereby recovering ammonia. This treatment has the advantage of avoiding the release of ammonia gas into the atmosphere.

In a third aspect the invention provides a method for removing sulphate in anaerobic digesters, the method comprising: submerging in anaerobic digesters a submergible bio-electrochemical system according to the first aspect of the invention; operating the bio-electrochemical system, thereby producing electricity and collecting a fluid effluent out of the second fluid outlet of said acid recovery section.

In the method for removing sulphate in anaerobic digesters according to the third aspect of the invention the operating may comprise: introducing a fluid, such as water, into the second fluid inlet of the acid recovery section; introducing a waste fluid at the inlet of the anode chamber, such as waste influent to the anaerobic digester or the waste effluent at the outlet of the anaerobic digester; introducing a stream of fluid at the inlet of the cathode chamber, such as air or a gas mixture containing oxygen gas; collecting an anode chamber effluent of treated waste fluid at the outlet of the anode chamber; collecting an acid recovery section effluent of a solution of sulphuric acid or hydrochloric acid at the second fluid outlet of the acid recovery section.

In a further aspect the invention provides a method for removing ammonia and sulphate in anaerobic digesters, the method comprising: submerging in anaerobic digesters a submergible bio-electrochemical system according to the first aspect of the invention; operating the bio-electrochemical system, thereby producing electricity and a stream of air and ammonia gas out of the fluid outlet of the cathode chamber and collecting a fluid effluent out of the second fluid outlet of the acid recovery section.

In the method for removing ammonia and sulphate in anaerobic digesters according to the further aspect of the invention, the operating may comprise: introducing a fluid, such as water, into the second fluid inlet of the acid recovery section; introducing a waste fluid at the inlet of the anode chamber, such as waste influent to the anaerobic digester or waste effluent at the outlet of the anaerobic digester; introducing a stream of fluid at the inlet of the cathode chamber, such as air or a gas mixture containing oxygen gas; collecting an anode chamber effluent of treated waste fluid at the outlet of the anode chamber; collecting a cathode chamber effluent of air or a gas mixture containing ammonia gas at the outlet of the cathode chamber; collecting an acid recovery section effluent of a solution of sulphuric acid or hydrochloric acid at the second fluid outlet of the acid recovery section.

In a fourth aspect the invention provides a plant for producing biogas comprising a submergible bio-electrochemical system according to the first aspect of the invention.

In a fifth aspect, the invention provides a power plant for producing electricity comprising a submergible bio-electrochemical system according to the first aspect of the invention.

The first, second, third and further aspects and embodiments of the present invention may each be combined with any of the other aspects and embodiments. These and other aspects of the invention will be apparent from and elucidated with reference to the embodiments described hereinafter.

### BRIEF DESCRIPTION OF THE FIGURES

The submergible bio-electrochemical system according to the invention will now be described in more detail with regard to the accompanying figures. The figures show one way of implementing the present invention and is not to be construed as being limiting to other possible embodiments falling within the scope of the attached claim set.
Figure 1 is a schematic illustration of the bio-electrochemical system according to some embodiments of the invention.
Figure 2 is a schematic illustration of the bio-electrochemical system according to some embodiments of the invention when submerged into an anaerobic digester.
Figure 3 is a schematic illustration of the bio-electrochemical system wherein the anode chamber comprises an acid recovery section according to some embodiments of the invention.
Figure 4 is a schematic illustration of a biogas production plant comprising the bio-electrochemical system according to some embodiments of the invention.
Figure 5 is a flow-chart of a method for removing ammonia according to some aspects of the invention.
Figure 6 is a flow-chart of a method for removing sulphuric acid according to some other aspects of the invention.

### DETAILED DESCRIPTION OF AN EMBODIMENT

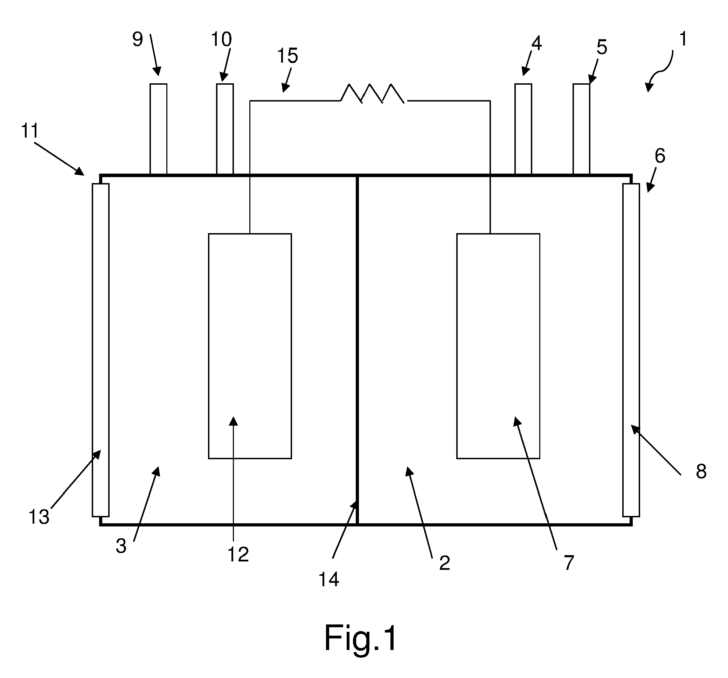

Figure 1 is a schematic illustration of the bio-electrochemical system 1 according to some embodiments of the invention.
The bio-electrochemical system 1 comprises an anode chamber 3 and a cathode chamber 2. The cathode chamber 2 comprises a fluid inlet 4, a fluid outlet 5, an opening 6 and a cathode 7. A cation exchange membrane (CEM) 8 is located at the opening 6 of the cathode chamber 2.
The anode chamber 3 comprises a fluid inlet 9, a fluid outlet 10, an opening 11 and an anode 12. An anion exchange membrane (AEM) 13 is located at the opening 11 of the anode chamber 3.

Figure 1 shows the bio-electrochemical system where the anode and cathode chambers 3 and 2 are compartments of a single bio-electrochemical chamber and the compartments are separated by fluid tight separation means such as a polycarbonate plate 14.
This separation means 14 allows keeping separated the two different chamber environments, i.e. anaerobic in the anode chamber 3 and aerobic in the cathode chamber 2.
The anode 12 and cathode 7 are connected through an external circuit 15. Thus the electrons produced at the anode 12 can reach the cathode 7 producing electricity when the bio-electrochemical system 1 is in operation.

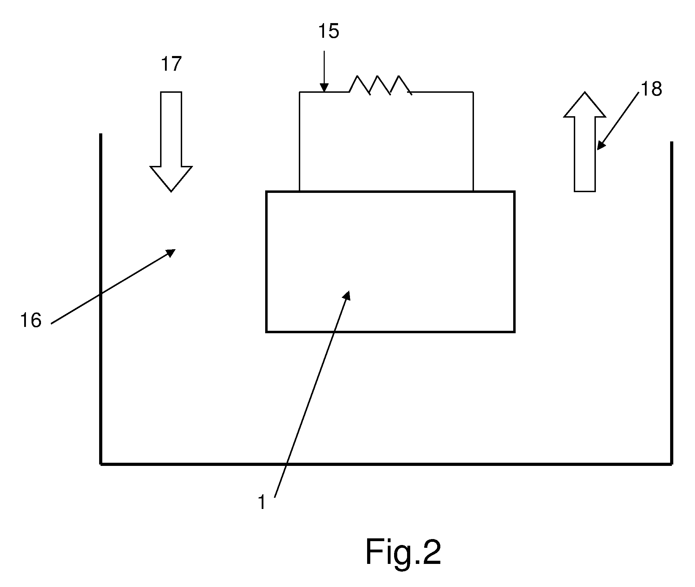

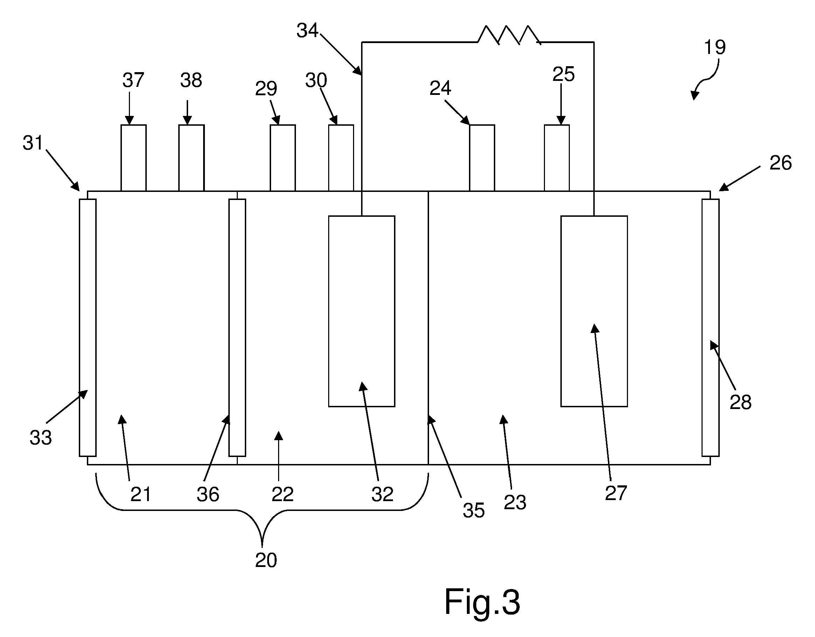

When in operation the bio-electrochemical system 1 is submerged into an anaerobic digester 16 as shown in figure 2. When submerged the openings 11 and 6 comprised in the anode chamber 3 and cathode chamber 2 allow the contact between the liquid contained in the anaerobic digester 16 and the AEM 13 and the CEM 8 respectively located at the opening 11 and opening 6. This allows for ion exchange between the liquid contained in the anaerobic digester 16 and the fluid introduced in the anode chamber 3 and the cathode chamber 2.
When in operation a waste fluid is introduced at the inlet 9 of the anode chamber 3. The waste fluid may be for example a waste influent 17 to the anaerobic digester 16 or waste effluent 18 at the outlet of the anaerobic digester 16.
A stream of fluid at the inlet 4 of the cathode chamber 2, is also introduced. The stream of fluid may be air or a gas mixture containing oxygen gas.
The bio-electrochemical system 1 then produces electricity by oxidizing organic material at the anode 12, thus producing electrons at the anode 12 which are collected through the external circuit 15 and reach the cathode 7, where they enter the reduction of oxygen leading to the formation of hydroxyl anions.
The effluent of the anode chamber 3 consisting of treated waste fluid is then collected at the outlet 10 of the anode chamber 3. The effluent of air or a gas mixture containing ammonia gas is collected at the outlet 5 of the cathode chamber 2. The effluent of air or a gas mixture containing ammonia gas may also be referred to as cathode effluent and may be treated separately with acidic solution so as to neutralize the ammonia present in the effluent. In some embodiments according to figure 3, this acid may be directly recovered from the liquid contained in the anaerobic digester.

Figure 3 is a schematic illustration of the bio-electrochemical system 19 wherein the anode chamber 20 comprises an acid recovery section 21 and an anode section 22 according to some embodiments of the invention.
The bio-electrochemical system 19 as shown in figure 3 comprises an anode chamber 20 and a cathode chamber 23. The cathode chamber 23 comprises a fluid inlet 24, a fluid outlet 25, an opening 26 and a cathode 27. A cation exchange membrane (CEM) 28 is located at the opening 26 of the cathode chamber 23.
The anode chamber 20 comprises a fluid inlet 29, a fluid outlet 30, an opening 31 and an anode 32. An anion exchange membrane (AEM) 33 is located at the opening 31 of the anode chamber 20. The anode and cathode chambers 20 and 23 are separated by fluid tight separation means such as a polycarbonate plate 35.
The anode 32 and cathode 27 are connected through an external circuit 34. Thus the electrons produced at the anode can reach the cathode producing electricity when the bio-electrochemical system 19 is in operation.
The bio-electrochemical system 19 is characterized by the separation of the anode chamber 20 into two sections: the acid recovery section 21 delimited by the opening 31 and a membrane 36 and an anode section 22 that comprises the anode 32 and is delimited by the membrane 36 and the polycarbonate plate 35. The membrane 36 may be a dipolar membrane or a further CEM.
The acid recovery section 21 further comprises a second fluid inlet 37 and a second fluid outlet 38.

When in operation the bio-electrochemical system 19 is submerged into an anaerobic digester as shown in figure 2 for bio-electrochemical system 1. When submerged the openings 31 and 26 comprised in the anode chamber 20 and cathode chamber 23 allow the contact between the liquid contained in the anaerobic digester and the AEM 33 and the CEM 28 respectively located at the opening 31 and opening 26. This allows for ion exchange between the liquid contained in the anaerobic digester and the fluid introduced in the anode chamber 20 and the cathode chamber 23.
When in operation a fluid, such as water, is introduced into the second fluid inlet 37 of the acid recovery section 21.

A waste fluid is then introduced at the inlet 29 of the anode chamber 20. The waste fluid may be, for example, a waste influent 17 to the anaerobic digester 16 or waste effluent 18 at the outlet of the anaerobic digester 16.
A stream of fluid at the inlet 24 of the cathode chamber 23 is also introduced. The stream of fluid may be air or a gas mixture containing oxygen gas.
The bio-electrochemical system 19 then produces electricity by oxidizing organic material at the anode 32, thus producing electrons at the anode 32 which are collected through the external circuit 34 and reach the cathode 27, where they enter the reduction of oxygen leading to the formation of hydroxyl anions.
The effluent of the anode chamber 20 consisting of treated waste fluid is then collected at the outlet 30 of the anode chamber 20. The effluent of air or a gas mixture containing ammonia gas is collected at the outlet 25 of the cathode chamber 23.
The effluent of the acid recovery section 21 is an acidic solution, e.g. a sulphuric
acid aqueous solution and is collected at the outlet 38 of the acid recovery section 21. In some embodiments the acidic solution at the outlet 38 may be directly used to neutralise the effluent of air or a gas mixture containing ammonia gas collected at the outlet 25 of the cathode chamber 23.

The anode 32 or 12 may comprise anaerobic microorganisms. In this case as mentioned above the microorganisms can oxidize various types of organic matter, e.g. present in the effluent from anaerobic digester and transfer electrons to the anode. At the cathode 27 or 7 oxygen is reduced by accepting the transferred electrons. The chemical reactions involved are shown as follows if acetate is the organic matter that is oxidized at the anode:

CH₃COO⁻+ 4H₂O → 2HCO₃⁻+8e⁻+9H⁺ [1]

O₂+4e⁻ +2H₂O → 4OH⁻ [2]

When electrical current is produced through the microorganisms oxidation of the organic matter at the anode and the oxygen reduction at the cathode, ammonium cations NH₄⁺ migrate into the cathode chamber and by reacting with the produced OH⁻ leads to the production of ammonia.

When the acid recovery section is present, anions, such as SO₄²⁻ or Cl⁻ migrate into the acid recovery section to alleviate the ionic imbalances. Meanwhile, protons (H⁺) in the anode chamber also migrate into the acid recovery chamber due to ionic imbalances leading to the formation of an acidic solution in the acid recovery section and therefore reducing the pH value in the acid recovery section.

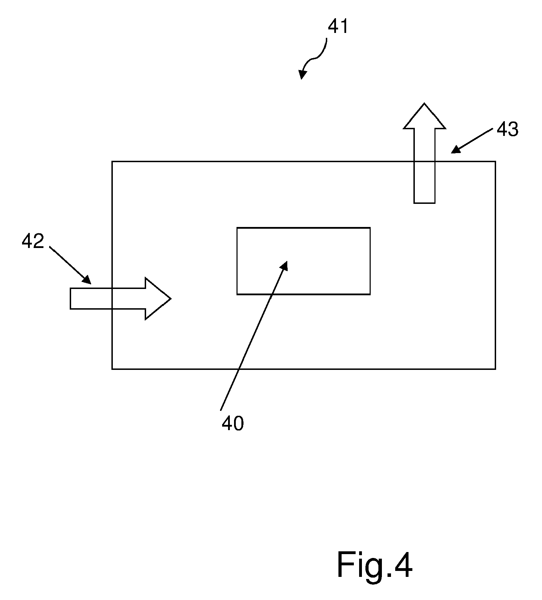

Figure 4 is a schematic illustration of a biogas production plant 41 comprising the bio-electrochemical system 40 according to some embodiments of the invention, showing a waste influent 42 and a biogas effluent 43.

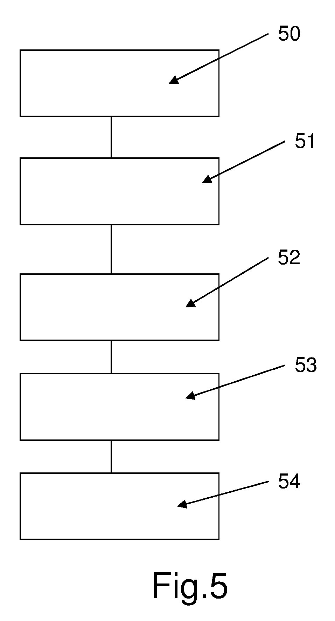

Figure 5 is a flow-chart of a method for removing ammonia according to some aspects of the invention. The method for removing ammonia in anaerobic digesters comprises the steps of:
- submerging 50 in anaerobic digesters a submergible bio-electrochemical system according to the first aspect of the invention;
- introducing 51 a waste fluid at the inlet of the anode chamber;
- introducing 52 a stream of fluid at the inlet of the cathode chamber;
- collecting 53 an anode chamber effluent of treated waste fluid at the outlet of the anode chamber;
- collecting 54 a cathode chamber effluent of air or a gas mixture containing ammonia gas at the outlet of the cathode chamber.

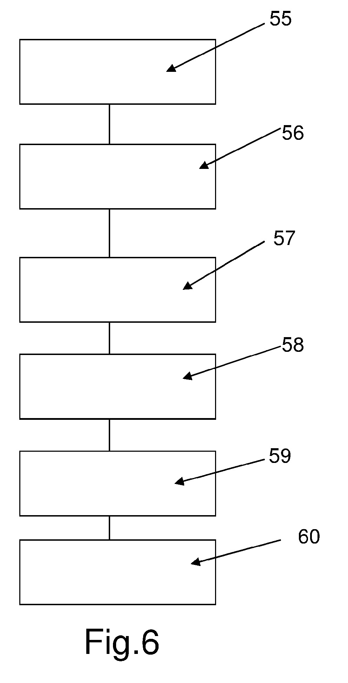

Figure 6 is a flow-chart of a method for removing sulphuric acid according to some other aspects of the invention. The method for removing sulphuric acid in anaerobic digesters comprises the steps of:
- submerging 55 in anaerobic digesters a submergible bio-electrochemical system according to the first aspect of the invention;
- introducing 56 a fluid, such as water, into the second fluid inlet of the acid recovery section
   - - introducing 57 a waste fluid at the inlet of the anode chamber;
   - - introducing 58 a stream of fluid at the inlet of the cathode chamber;
   - - collecting 59 an anode chamber effluent of treated waste fluid at the outlet of the anode chamber;
   - - collecting 60 an acid recovery section effluent of a solution of sulphuric acid at the second fluid outlet of the acid recovery section.

In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention.

## Claims

1. A submergible bio-electrochemical system for recovering ammonia in an anaerobic digester by producing electricity and a stream of air and ammonia gas out of said system, said system comprising:
- an anode chamber;
- a cathode chamber;
wherein said cathode chamber comprises a fluid inlet, a fluid outlet, an opening, a cathode and a cation exchange membrane (CEM) located at said opening of said cathode chamber, and wherein said anode chamber comprises a fluid inlet, a fluid outlet, an opening, an anode wherein said anode comprises anaerobic microorganisms and an anion exchange membrane (AEM) located at said opening of said anode chamber.

2. A submergible bio-electrochemical system according to claim 1, wherein when submerged, said opening comprised in said anode chamber allows for contact between the liquid contained in said anaerobic digester and said AEM contained in said anode chamber.

3. A submergible bio-electrochemical system according to any of the preceding claims, wherein when submerged said opening comprised in said cathode chamber allows for contact between the liquid contained in said anaerobic digester and said CEM contained in said cathode chamber.

4. A submergible bio-electrochemical system according to any of the preceding claims, wherein said anode and cathode chamber are mechanically joined to each other.

5. A submergible bio-electrochemical system according to any of the preceding claims 1-3, wherein said anode and cathode chamber are separated from each other.

6. A submergible bio-electrochemical system according to any of the preceding claims, wherein said anode and cathode chambers are compartments of a single bio-electrochemical chamber, said compartments being separated by fluid tight separation means.

7. A submergible bio-electrochemical system according to any of the preceding claims, wherein said anode chamber is separated into two sections, an acid recovery section and an anode section comprising said anode, wherein said acid recovery section comprises said opening of said anode chamber and is separated from said anode section by a membrane.

8. A submergible bio-electrochemical system according to claim 7, wherein said acid recovery section further comprises a second fluid inlet and a second fluid outlet.

9. A method for removing ammonia in anaerobic digesters, said method comprising:
- submerging in anaerobic digesters a submergible bio-electrochemical system according to any of the preceding claims 1-6;
- operating said bio-electrochemical system, thereby producing electricity and a stream of air and ammonia gas out of said fluid outlet of said cathode chamber.

10. A method for removing ammonia in anaerobic digesters according to claim 9, wherein operating comprises:
- introducing a waste fluid at the inlet of the anode chamber, such as waste influent to said anaerobic digester or waste effluent at the outlet of the anaerobic digester;
- introducing a stream of fluid at the inlet of the cathode chamber, such as air or a gas mixture containing oxygen gas;
- collecting an anode chamber effluent of treated waste fluid at the outlet of the anode chamber;
- collecting a cathode chamber effluent of air or a gas mixture containing ammonia gas at the outlet of the cathode chamber.

11. A method for removing ammonia in anaerobic digesters according to claim 10, wherein operating further comprises:
- treating said cathode chamber effluent by bubbling said cathode chamber effluent though an acid solution thereby recovering ammonia.

12. A method for removing ammonia and sulphate in anaerobic digesters, said method comprising:
- submerging in anaerobic digesters a submergible bio-electrochemical system according to any of the preceding claims 7-8;
- operating said bio-electrochemical system, thereby producing electricity and a stream of air and ammonia gas out of said fluid outlet of said cathode chamber and collecting a fluid effluent out of said second fluid outlet of said acid recovery section.

13. A method for removing ammonia and sulphate in anaerobic digesters according to claim 12, wherein operating comprises:
- introducing a fluid, such as water, into said second fluid inlet of said acid recovery section;
- introducing a waste fluid at the inlet of the anode chamber, such as waste influent to said anaerobic digester or waste effluent at the outlet of the anaerobic digester;
- introducing a stream of fluid at the inlet of the cathode chamber, such as air or a gas mixture containing oxygen gas;
- collecting an anode chamber effluent of treated waste fluid at the outlet of the anode chamber;
- collecting a cathode chamber effluent of air or a gas mixture containing ammonia gas at the outlet of the cathode chamber;
- collecting an acid recovery section effluent of a solution of sulphuric acid or hydrochloric acid at the second fluid outlet of said acid recovery section.

14. A method for removing sulphate in anaerobic digesters, said method comprising:
- submerging in anaerobic digesters a submergible bio-electrochemical system according to any of the preceding claims 7-8;
- operating said bio-electrochemical system, thereby producing electricity and collecting a fluid effluent out of said second fluid outlet of said acid recovery section.

15. A method for removing sulphate in anaerobic digesters according to claim 14, wherein operating comprises:
- introducing a fluid, such as water, into said second fluid inlet of said acid recovery section;
- introducing a waste fluid at the inlet of the anode chamber, such as waste influent to said anaerobic digester or the waste effluent at the outlet of the anaerobic digester;
- introducing a stream of fluid at the inlet of the cathode chamber, such as air or a gas mixture containing oxygen gas;
- collecting an anode chamber effluent of treated waste fluid at the outlet of the anode chamber;
- collecting an acid recovery section effluent of a solution of sulphuric acid or hydrochloric acid at the second fluid outlet of said acid recovery section.

16. A plant for producing biogas comprising a submergible bio-electrochemical system according to any of the preceding claims 1-8.

17. A power plant for producing electricity comprising a submergible bio-electrochemical system according to any of the preceding claims 1-8.

## Patentansprüche

1. Eintauchbares bioelektrochemisches System zur Rückgewinnung von Ammoniak in einem anaeroben Faultank durch Erzeugen von elektrischem Strom und einem Strom aus Luft und Ammoniakgas aus dem System, wobei das System umfasst:
- eine Anodenkammer;
- eine Kathodenkammer;
wobei die Kathodenkammer einen Fluideinlass, einen Fluidauslass, eine Öffnung, eine Kathode und eine Kationen-Austauschmembran (CEM) an der Öffnung der Kathodenkammer umfasst und wobei die Anodenkammer einen Fluideinlass, einen Fluidauslass, eine Öffnung und eine Anode umfasst, wobei die Anode anaerobe Mikroorganismen und eine Anionen-Austauschmembran (AEM) an der Öffnung der Anodenkammer umfasst.

2. Eintauchbares bioelektrochemisches System nach Anspruch 1, wobei die an der Anodenkammer enthaltene Öffnung beim Eintauchen einen Kontakt zwischen dem in dem anaeroben Faulbehälter enthaltenen Fluid und der in der Anodenkammer enthaltenen AEM zulässt.

3. Eintauchbares bioelektrochemisches System nach einem der vorhergehenden Ansprüche, wobei die in der Kathodenkammer enthaltene Öffnung beim Eintauchen einen Kontakt zwischen dem in dem anaeroben Faulbehälter enthaltenen Fluid und der in der Kathodenkammer enthaltenen CEM zulässt.

4. Eintauchbares bioelektrochemisches System nach einem der vorhergehenden Ansprüche, wobei die Anoden- und die Kathodenkammer mechanisch miteinander verbunden sind.

5. Eintauchbares bioelektrochemisches System nach einem der vorhergehenden Ansprüche 1-3, wobei die Anoden- und die Kathodenkammer getrennt voneinander sind.

6. Eintauchbares bioelektrochemisches System nach einem der vorhergehenden Ansprüche, wobei die Anoden- und die Kathodenkammer Räume einer einzigen bioelektrochemischen Kammer sind, wobei die Räume durch fluiddichte Trennungsmittel voneinander getrennt sind.

7. Eintauchbares bioelektrochemisches System nach einem der vorhergehenden Ansprüche, wobei die Anoden- und die Kathodenkammer in zwei Abschnitte unterteilt sind, wobei die Anode einen Säurerückgewinnungsabschnitt und einen Anodenabschnitt umfasst, wobei der Säurerückgewinnungsabschnitt die Öffnung der Anodenkammer umfasst und von dem Anodenabschnitt durch eine Membran getrennt ist.

8. Eintauchbares bioelektrochemisches System nach Anspruch 7, wobei der Säurerückgewinnungsabschnitt weiterhin einen zweiten Fluideinlass und einen zweiten Fluidauslass umfasst.

9. Verfahren zum Entfernen von Ammoniak in anaeroben Faulbehältern, wobei das Verfahren umfasst:
- Eintauchen eines eintauchbaren bioelektrochemischen Systems nach einem der vorhergehenden Ansprüche 1-6 in anaerobe Faulbehälter;
- Betreiben des bioelektrochemischen Systems, wodurch elektrischer Strom und ein Strom aus Luft und Ammoniakgas aus dem Fluidauslass der Kathodenkammer erzeugt werden.

10. Verfahren zum Entfernen von Ammoniak in anaeroben Faulbehältern nach Anspruch 9, wobei das Betreiben umfasst:
- Einführen von Fluidabfall an dem Einlass der Anodenkammer, wie Zulaufabfall an dem anaeroben Faulbehälter oder Ablaufabfall an dem Auslass des anaeroben Faulbehälters;
- Einführen eines Fluidstroms am Einlass der Kathodenkammer, wie Luft oder einer Gasmischung mit Sauerstoffgas;
- Sammeln eines Ablaufs aus aufbereitetem Fluidabfall aus der Anodenkammer am Auslass der Anodenkammer;
- Sammeln eines Ablaufs aus Ammoniakgas enthaltender Luft oder Ammoniakgas enthaltendem Gasgemisch aus der Kathodenkammer am Auslass der Kathodenkammer.

11. Verfahren zum Entfernen von Ammoniak in anaeroben Faulbehältern nach Anspruch 10, wobei das Betreiben weiterhin umfasst:
- Behandeln des Ablaufs der Kathodenkammer, indem der Ablauf der Kathodenkammer durch eine Säurelösung geperlt wird, wodurch der Ammoniak zurückgewonnen wird.

12. Verfahren zum Entfernen von Ammoniak und Sulfat in anaeroben Faulbehältern, wobei das Verfahren umfasst:
- Eintauchen eines eintauchbaren bioelektrochemischen Systems nach einem der vorhergehenden Ansprüche 7-8 in anaerobe Faulbehälter;
- Betreiben des bioelektrochemischen Systems, wodurch elektrischer Strom und ein Strom aus Luft und Ammoniakgas aus dem Fluidauslass der Kathodenkammer erzeugt werden, und Sammeln eines Fluidablaufs aus dem zweiten Fluidauslass des Säurerückgewinnungsabschnitts.

13. Verfahren zum Entfernen von Ammoniak und Sulfat in anaeroben Faulbehältern nach Anspruch 12, wobei das Betreiben umfasst:
- Einführen eines Fluids, wie Wasser, in den zweiten Fluideinlass des Säurerückgewinnungsabschnitts;
- Einführen von Fluidabfall an dem Einlass der Anodenkammer, wie Zulaufabfall an dem anaeroben Faulbehälter oder Ablaufabfall an dem Auslass des anaeroben Faulbehälters;
- Einführen eines Fluidstroms am Einlass der Kathodenkammer, wie Luft oder einer Gasmischung mit Sauerstoffgas;
- Sammeln eines Ablaufs aus aufbereitetem Fluidabfall aus der Anodenkammer am Auslass der Anodenkammer;
- Sammeln eines Ablaufs aus Ammoniakgas enthaltender Luft oder Ammoniakgas enthaltendem Gasgemisch aus der Kathodenkammer am Auslass der Kathodenkammer;
- Sammeln eines Ablaufs aus einer Lösung aus Schwefelsäure oder Salzsäure aus dem Säurerückgewinnungsabschnitt am zweiten Fluidauslass des Säurerückgewinnungsabschnitts.

14. Verfahren zum Entfernen von Sulfat in anaeroben Faulbehältern, wobei das Verfahren umfasst:
- Eintauchen eines eintauchbaren bioelektrochemischen Systems nach einem der vorhergehenden Ansprüche 7-8 in anaerobe Faulbehälter;
- Betreiben des bioelektrochemischen Systems, wodurch elektrischer Strom erzeugt wird, und Sammeln eines Fluidablaufs aus dem zweiten Fluidauslass des Säurerückgewinnungsabschnitts.

15. Verfahren zum Entfernen von Sulfat in anaeroben Faulbehältern nach Anspruch 14, wobei das Betreiben umfasst:
- Einführen eines Fluids, wie Wasser, in den zweiten Fluideinlass des Säurerückgewinnungsabschnitts;
- Einführen von Fluidabfall an dem Einlass der Anodenkammer, wie Zulaufabfall an dem anaeroben Faulbehälter oder Ablaufabfall an dem Auslass des anaeroben Faulbehälters;
- Einführen eines Fluidstroms am Einlass der Kathodenkammer, wie Luft oder einer Gasmischung mit Sauerstoffgas;
- Sammeln eines Ablaufs aus aufbereitetem Fluidabfall aus der Anodenkammer am Auslass der Anodenkammer;
- Sammeln eines Ablaufs aus einer Lösung aus Schwefelsäure oder Salzsäure aus dem Säurerückgewinnungsabschnitt am zweiten Fluidauslass des Säurerückgewinnungsabschnitts.

16. Anlage zum Erzeugen von Biogas, umfassend ein eintauchbares bioelektrochemisches System nach einem der vorhergehenden Ansprüche 1-8.

17. Kraftwerk zum Erzeugen von Strom, umfassend ein eintauchbares bioelektrochemisches System nach einem der vorhergehenden Ansprüche 1-8.

## Revendications

1. Système bioélectrochimique immergeable pour retirer de l'ammoniaque dans un digesteur anaérobie en produisant de l'électricité et un flux d'air et de gaz ammoniac sortant dudit système, ledit système comprenant :
- une chambre anodique ;
- une chambre cathodique ;
dans lequel ladite chambre cathodique comprend une entrée de fluide, une sortie de fluide, une ouverture, une cathode et une membrane échangeuse de cations (MEC) située au niveau de ladite ouverture de ladite chambre cathodique, et dans lequel ladite chambre anodique comprend une entrée de fluide, une sortie de fluide, une ouverture, une anode dans laquelle ladite anode comprend des micro-organismes anaérobies et une membrane échangeuse d'anions (MEA) située au niveau de ladite ouverture de ladite chambre anodique.

2. Système bioélectrochimique immergeable selon la revendication 1, dans lequel, lorsqu'il est immergé, ladite ouverture comprise dans ladite chambre anodique permet le contact entre le liquide contenu dans ledit digesteur anaérobie et ladite MEA contenue dans ladite chambre anodique.

3. Système bioélectrochimique immergeable selon l'une quelconque des revendications précédentes, dans lequel, lorsqu'il est immergé, ladite ouverture comprise dans ladite chambre cathodique permet le contact entre le liquide contenu dans ledit digesteur anaérobie et ladite MEC contenue dans ladite chambre cathodique.

4. Système bioélectrochimique immergeable selon l'une quelconque des revendications précédentes, dans lequel lesdites chambres anodique et cathodique sont reliées l'une à l'autre mécaniquement.

5. Système bioélectrochimique immergeable selon l'une quelconque des revendications 1 à 3, dans lequel lesdites chambres anodique et cathodique sont séparées l'une de l'autre.

6. Système bioélectrochimique immergeable selon l'une quelconque des revendications précédentes, dans lequel lesdites chambres anodique et cathodique sont des compartiments d'une même chambre bioélectrochimique, lesdits compartiments étant séparés par un moyen de séparation étanche aux fluides.

7. Système bioélectrochimique immergeable selon l'une quelconque des revendications précédentes, dans lequel ladite chambre anodique est séparée en deux parties, une partie de récupération d'acide et une partie anodique comprenant ladite anode, dans lequel ladite partie de récupération d'acide comprend ladite ouverture de ladite chambre anodique et est séparée de ladite partie anodique par une membrane.

8. Système bioélectrochimique immergeable selon la revendication 7, dans lequel ladite partie de récupération d'acide comprend également une deuxième entrée de fluide et une deuxième sortie de fluide.

9. Procédé pour retirer de l'ammoniaque dans des digesteurs anaérobies, ledit procédé comprenant :
- l'immersion dans des digesteurs anaérobies d'un système bioélectrochimique immergeable selon l'une quelconque des revendications 1 à 6 ;
- le fonctionnement dudit système bioélectrochimique, en produisant ainsi de l'électricité et un flux d'air et de gaz ammoniac sortant de ladite sortie de fluide de ladite chambre cathodique.

10. Procédé pour retirer de l'ammoniaque dans des digesteurs anaérobies selon la revendication 9, dans lequel le fonctionnement comprend :
- l'introduction d'un fluide résiduaire au niveau de l'entrée de la chambre anodique, tel que l'influent résiduaire pénétrant dans ledit digesteur anaérobie ou l'effluent résiduaire à la sortie du digesteur anaérobie ;
- l'introduction d'un flux de fluide au niveau de l'entrée de la chambre cathodique, tel que de l'air ou un mélange de gaz comprenant de l'oxygène gazeux ;
- la collecte d'un effluent de la chambre anodique, composé d'un fluide résiduaire traité, au niveau de la sortie de la chambre anodique ;
- la collecte d'un effluent de la chambre cathodique, composé d'air ou d'un mélange de gaz contenant du gaz ammoniac, au niveau de la sortie de la chambre cathodique.

11. Procédé pour retirer de l'ammoniaque dans des digesteurs anaérobies selon la revendication 10, dans lequel le fonctionnement comprend également :
- le traitement dudit effluent de la chambre cathodique par barbotage dudit effluent de la chambre cathodique au travers d'une solution acide en récupérant ainsi de l'ammoniaque.

12. Procédé pour retirer de l'ammoniaque et du sulfate dans des digesteurs anaérobies, ledit procédé comprenant :
- l'immersion dans des digesteurs anaérobies d'un système bioélectrochimique immergeable selon l'une quelconque des revendications 7 à 8 ;
- le fonctionnement dudit système bioélectrochimique, en produisant ainsi de l'électricité et un flux d'air et de gaz ammoniac sortant de ladite sortie de fluide de ladite chambre cathodique et la collecte d'un effluent fluide provenant de ladite deuxième sortie de fluide de ladite partie de récupération d'acide.

13. Procédé pour retirer de l'ammoniaque et du sulfate dans des digesteurs anaérobies selon la revendication 12, dans lequel le fonctionnement comprend :
- l'introduction d'un fluide, tel que de l'eau, dans ladite deuxième entrée de fluide de ladite partie de récupération d'acide ;
- l'introduction d'un fluide résiduaire au niveau de l'entrée de la chambre anodique, tel que l'influent résiduaire pénétrant dans ledit digesteur anaérobie ou l'effluent résiduaire à la sortie du digesteur anaérobie ;
- l'introduction d'un flux de fluide au niveau de l'entrée de la chambre cathodique, tel que de l'air ou un mélange de gaz comprenant de l'oxygène gazeux ;
- la collecte d'un effluent de la chambre anodique, composé d'un fluide résiduaire traité, au niveau de la sortie de la chambre anodique ;
- la collecte d'un effluent de la chambre cathodique, composé d'air ou d'un mélange de gaz contenant du gaz ammoniac, au niveau de la sortie de la chambre cathodique ;
- la collecte d'un effluent de la partie de récupération d'acide, composé d'une solution d'acide sulfurique ou d'acide chlorhydrique, au niveau de la deuxième sortie de fluide de ladite partie de récupération d'acide.

14. Procédé pour retirer du sulfate dans des digesteurs anaérobies, ledit procédé comprenant :
- l'immersion dans des digesteurs anaérobies d'un système bioélectrochimique immergeable selon l'une quelconque des revendications 7 à 8 ;
- le fonctionnement dudit système bioélectrochimique, en produisant ainsi de l'électricité et en recueillant un effluent fluide provenant de ladite deuxième sortie de fluide de ladite partie de récupération d'acide.

15. Procédé pour retirer du sulfate dans des digesteurs anaérobies selon la revendication 14, dans lequel le fonctionnement comprend :
- l'introduction d'un fluide, tel que de l'eau, dans ladite deuxième entrée de fluide de ladite partie de récupération d'acide ;
- l'introduction d'un fluide résiduaire au niveau de l'entrée de la chambre anodique, tel que l'influent résiduaire pénétrant dans ledit digesteur anaérobie ou l'effluent résiduaire à la sortie du digesteur anaérobie ;
- l'introduction d'un flux de fluide au niveau de l'entrée de la chambre cathodique, tel que de l'air ou un mélange de gaz comprenant de l'oxygène gazeux ;
- la collecte d'un effluent de la chambre anodique, composé d'un fluide résiduaire traité, au niveau de la sortie de la chambre anodique ;
- la collecte d'un effluent de la partie de récupération d'acide, composé d'une solution d'acide sulfurique ou d'acide chlorhydrique, au niveau de la deuxième sortie de fluide de ladite partie de récupération d'acide.

16. Usine de production de biogaz comprenant un système bioélectrochimique immergeable selon l'une quelconque des revendications 1 à 8.

17. Usine de production d'électricité comprenant un système bioélectrochimique immergeable selon l'une quelconque des revendications 1 à 8.
